# EUROPEAN PATENT APPLICATION

(11) **EP 4 400 512 A1**
(43) Date of publication of application: **17.07.2024**
(21) Application number: 21956409.3
(22) Date of filing: 10.09.2021
(51) Int. Cl.: C07K 16/22, A61K 39/395, A61P 35/00, C12N 5/10, C12N 15/13

(54) **ANTI-ANG2 ANTIBODY, PREPARATION METHOD THEREFOR, AND APPLICATION THEREOF**

(71) Applicant: Soter Biopharma Pte. Ltd., Singapore 179803 (SG)
(72) Inventor: SHI, Jian, Wuhan, Hubei 430075 (CN); FANG, Lijuan, Wuhan, Hubei 430075 (CN); YAN, Yongxiang, Wuhan, Hubei 430075 (CN); ZHANG, Jing, Wuhan, Hubei 430075 (CN); HUA, Shan, Wuhan, Hubei 430075 (CN); ZHOU, Pengfei, Wuhan, Hubei 430075 (CN)
(74) Representative: Croce, Valeria
(86) International application number: PCT/CN2021/117697
(87) International publication number: WO 2023/035226

(57) **Abstract**

The present invention relates to an anti-ANG2 heavy-chain single-domain antibody, and an application thereof.

## Description

### TECHNICAL FIELD

The present disclosure relates to the field of medicines and, specifically, to an ANG2 (human angiopoietin-2) antibody, a preparation method therefor and an application thereof.

### BACKGROUND

An antibody targeting ANG2 can inhibit neovascular generation and leakage and reduce the occurrence of an inflammatory response. ANG/Tie2 is an important signal pathway that regulates angiogenesis. ANG1 promotes the phosphorylation of an endothelial cell receptor Tie2, attracts perivascular cells such as vascular smooth muscle cells and pericytes to surround and support endothelial cells, promotes vascular remodeling, maintains vascular integrity and regulates a vascular function. ANG1 can protect a blood vessel from a lesion and maintain vascular morphology, while ANG2 promotes vascular leakage, resulting in hypotension and an abnormal vascular structure. Although ANG2 antibodies developed by many enterprises are subjected to clinical trials, there is no antibody drug targeting ANG2 on the market at present.

### SUMMARY

The present disclosure provides a specific monoclonal antibody targeting ANG2 that plays a role in inhibiting pro-angiogenesis activity of ANG2 and treating diseases and conditions caused by or related to an angiogenic process such as fundus neovascular disease, rheumatoid arthritis and psoriasis. In addition, angiogenesis is very critical to tumor growth and maintenance, and the monoclonal antibody that inhibits ANG2 can also play a role in a process of treating a cancer. Furthermore, the present disclosure further provides a bispecific antibody targeting ANG2 and VEGF. The antibody of the present disclosure can bind to human ANG2, rabbit ANG2 and monkey ANG2.

Specifically, the present disclosure relates to the following aspects:
1. An anti-ANG2 heavy-chain single-domain antibody, comprising HCDR1, HCDR2 and HCDR3 contained in a heavy chain variable region shown in any one of SEQ ID NOs: 1 to 10 or variants of HCDR1, HCDR2 and HCDR3,
   preferably, according to an IMGT numbering system, comprising HCDR1, HCDR2 and HCDR3 selected from a group consisting of:
   (1) HCDR1 shown in SEQ ID NO: 21, HCDR2 shown in SEQ ID NO: 22 and HCDR3 shown in SEQ ID NO: 23,
   (2) HCDR1 shown in SEQ ID NO: 34, HCDR2 shown in SEQ ID NO: 35 and HCDR3 shown in SEQ ID NO: 36,
   (3) HCDR1 shown in SEQ ID NO: 37, HCDR2 shown in SEQ ID NO: 38 and HCDR3 shown in SEQ ID NO: 39,
   (4) HCDR1 shown in SEQ ID NO: 40, HCDR2 shown in SEQ ID NO: 41 and HCDR3 shown in SEQ ID NO: 42, or variants of HCDR1 shown in SEQ ID NO: 40, HCDR2 shown in SEQ ID NO: 41 and HCDR3 shown in SEQ ID NO: 42, and a combination thereof, wherein the variants each have one or more (preferably one, two or three) conservative amino acid mutations (preferably a substitution, an insertion or a deletion) compared with HCDR1 shown in SEQ ID NO: 40, HCDR2 shown in SEQ ID NO: 41 and HCDR3 shown in SEQ ID NO: 42, respectively, and an amino acid sequence that has an affinity for binding to ANG2 is retained,
      preferably, HCDR1 shown in SEQ ID NO: 40, HCDR2 shown in SEQ ID NO: 41 and HCDR3 shown in SEQ ID NO: 42, wherein amino acids at positions 5 to 8 and positions 10 and 11 in CDR1 shown in SEQ ID NO: 40, amino acids at positions 2 to 5 and position 7 in CDR2 shown in SEQ ID NO: 41 and amino acids at position 1 and positions 3 to 8 in CDR3 shown in SEQ ID NO: 42 are selected from an amino acid X, and the amino acid X is selected from a group consisting of Ala, Arg, Asn, Asp, Cys, Gln, Glu, Gly, His, Ile, Leu, Lys, Met, Phe, Pro, Ser, Thr, Trp, Tyr and Val,
      preferably, a variant of HCDR1 shown in SEQ ID NO: 40, wherein glycine G at position 1 may be substituted by an amino acid selected from a group consisting of: Ala, Val, Leu and Ile; phenylalanine F at position 2 may be substituted by Tyr; proline P at position 3 may be substituted by an amino acid selected from a group consisting of: Trp and His; Ser at position 4 may be substituted by Thr; Ser at position 9 may be substituted by Thr; and Gln at position 12 may be substituted by Asn;
      a variant of HCDR2 shown in SEQ ID NO: 41, wherein Ile at position 1 may be substituted by an amino acid selected from a group consisting of: Ala, Val, leu and Gly; Leu at position 6 may be substituted by Ile; and Lys at position 8 may be substituted by Arg;
      a variant of HCDR3 shown in SEQ ID NO: 42, wherein Val at position 2 may be substituted by an amino acid selected from a group consisting of: Ala, Gly, Leu and Ile; and Asp at position 9 may be substituted by Glu,
   (5) HCDR1 shown in SEQ ID NO: 43, HCDR2 shown in SEQ ID NO: 44 and HCDR3 shown in SEQ ID NO: 45,
   (6) HCDR1 shown in SEQ ID NO: 46, HCDR2 shown in SEQ ID NO: 44 and HCDR3 shown in SEQ ID NO: 48,
   (7) HCDR1 shown in SEQ ID NO: 49, HCDR2 shown in SEQ ID NO: 44 and HCDR3 shown in SEQ ID NO: 48,
   (8) HCDR1 shown in SEQ ID NO: 52, HCDR2 shown in SEQ ID NO: 44 and HCDR3 shown in SEQ ID NO: 48, and
   (9) HCDR1 shown in SEQ ID NO: 55, HCDR2 shown in SEQ ID NO: 56 and HCDR3 shown in SEQ ID NO: 57.
2. The anti-ANG2 heavy-chain single-domain antibody according to item 1, wherein ANG2 is selected from human ANG2, rabbit ANG2 or monkey ANG2.
3. The anti-ANG2 heavy-chain single-domain antibody according to item 1 or 2, comprising a heavy chain variable region, wherein the heavy chain variable region comprises or consists of a sequence shown in any one of SEQ ID NOs: 1 to 10 or a sequence having at least 80%, 85%, 86%, 87%, 88%, 89%, 90%, 91%, 92%, 93%, 94%, 95%, 96%, 97%, 98% or 99% homology with the sequence shown in any one of SEQ ID NOs: 1 to 10 and having ANG2 binding activity.
4. A recombinant protein, comprising the anti-ANG2 heavy-chain single-domain antibody according to any one of items 1 to 3, preferably, the recombinant protein further comprises a bioactive substance, for example, a toxin having enzymatic activity or an active fragment (for example, abrin, ricin A, pseudomonas exotoxin or diphtheria toxin) of the toxin, a tumor necrosis factor or an interferon (for example, IFN-γ), a biological response modifier (for example, lymphokines IL-2, IL-2, IL2-6, IL-10 and GM-CSF) and/or an Fc fragment (for example, from an Fc region of IgG (for example, an IgG1, IgG2, IgG3 or IgG4 subtype), IgA1, IgA2, IgD, IgE or IgM, for example, from an Fc region of human, rabbit or monkey IgG, IgA1, IgD, IgE or IgM), preferably, the anti-ANG2 heavy-chain single-domain antibody and the Fc fragment are linked by a linker peptide, preferably, the linker peptide is (GGGGS)n, wherein n = 1, 2 or 3, preferably, the Fc fragment is a human IgG1 heavy chain constant region, more preferably an Fc fragment shown in GenBankNo. AK303185.1 or SEQ ID NO: 29, more preferably, the recombinant protein comprises or consists of sequences shown in SEQ ID NOs: 11 to 20.
5. A multi-specific antibody, preferably a bispecific antibody, comprising the anti-ANG2 heavy-chain single-domain antibody according to any one of items 1 to 3, preferably, a structure of the bispecific antibody comprises a light chain and a heavy chain, wherein the light chain and the heavy chain are paired to form an interchain disulfide bond, and two heavy chains are paired to form an interchain disulfide bond, wherein the heavy chain is (VH)-(CH1)-(hinge region)-(Fc)-(linker peptide)-(VHH), and the light chain is (VL)-(light chain constant region).
6. The multi-specific antibody according to item 5, wherein VHH is the anti-ANG2 single domain antibody according to any one of items 1 to 3, VH and VL are heavy chain and light chain variable regions of a second antibody, preferably, an antigen targeted by the second antibody is selected from an immune cell surface antigen, a tumor antigen, a virus, a bacterium, an endotoxin, a cytokine or a combination thereof; more preferably, the antigen targeted by the second antibody is selected from PD-L1, PD-1, VEGFA, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, GD3, GM2, RANKL, CD3 and/or CD16a, preferably, the antigen targeted by the second antibody is VEGF, more preferably, the second antibody is selected from an anti-VEGF antibody, the anti-VEGF antibody comprises HCDR1, HCDR2 and HCDR3 contained in a heavy chain variable region shown in SEQ ID NO: 24 (preferably, according to an IMGT numbering system, the anti-VEGF antibody comprises HCDR1 shown in SEQ ID NO: 50, HCDR2 shown in SEQ ID NO: 51 and HCDR3 shown in SEQ ID NO: 53) and LCDR1, LCDR2 and LCDR3 contained in a light chain variable region shown in SEQ ID NO: 27 (preferably, according to the IMGT numbering system, the anti-VEGF antibody comprises LCDR1 shown in SEQ ID NO: 54, LCDR2 shown in SEQ ID NO: 58 and LCDR3 shown in SEQ ID NO: 59), more preferably, a heavy chain variable region of the anti-VEGF antibody comprises or consists of a sequence shown in SEQ ID NO: 24 or a sequence having at least 80%, 85%, 90%, 91%, 92%, 93%, 94%, 95%, 96%, 97%, 98% or 99% homology with the sequence shown in SEQ ID NO: 24; and a light chain variable region of the anti-VEGF antibody comprises or consists of a sequence shown in SEQ ID NO: 27 or a sequence having at least 80%, 85%, 90%, 91%, 92%, 93%, 94%, 95%, 96%, 97%, 98% or 99% homology with the sequence shown in SEQ ID NO: 27.
7. The multi-specific antibody according to item 5 or 6, wherein a sequence of CH1 is shown in SEQ ID NO: 25, a sequence of the hinge region is shown in SEQ ID NO: 26, a sequence of the Fc constant region is shown in SEQ ID NO: 29, a sequence of the linker peptide is shown in SEQ ID NO: 30 or 47, a sequence of the light chain constant region is shown in SEQ ID NO: 28; preferably, the multi-specific antibody is selected from the following groups:
   (1) Y400C, which comprises or consists of a heavy chain and a light chain; wherein a sequence of the heavy chain comprises or consists of SEQ ID NOs: 24, 25, 26, 29, 30 and 4; and a sequence of the light chain comprises or consists of SEQ ID NOs: 27 and 28; and
   (2) Y400E, which comprises or consists of a heavy chain and a light chain, wherein a sequence of the heavy chain comprises or consists of SEQ ID NOs: 24, 25, 26, 29, 47 and 4; and a sequence of the light chain comprises or consists of SEQ ID NOs: 27 and 28.
8. A polynucleotide, encoding the anti-ANG2 heavy-chain single-domain antibody according to any one of items 1 to 3, the recombinant protein according to item 4 or the multi-specific antibody according to any one of items 5 to 7.
9. A vector, comprising the polynucleotide according to item 8.
10. A host cell, comprising the vector according to item 9.
11. A conjugate, comprising the anti-ANG2 heavy-chain single-domain antibody according to any one of items 1 to 3, the recombinant protein according to item 4, or the multi-specific antibody according to any one of items 5 to 7, and a conjugated moiety, wherein the conjugated moiety is a purification tag (for example, a His tag), a detectable label, a drug, a toxin, a cytokine, an enzyme or a combination thereof; preferably, the conjugated moiety is a radioisotope, a fluorescent substance, a chemiluminescent substance, a colored substance, a chemotherapeutic agent, a biological toxin, polyethylene glycol or an enzyme.
12. A kit, comprising the anti-ANG2 heavy-chain single-domain antibody according to any one of items 1 to 3, the recombinant protein according to item 4, the multi-specific antibody according to any one of items 5 to 7 or the conjugate according to item 11; preferably, the kit further comprises an anti-ANG2 heavy-chain single-domain antibody, a recombinant protein or a multi-specific antibody specifically recognizing the anti-ANG2 heavy-chain single-domain antibody according to any one of items 1 to 3, the recombinant protein according to item 4, the multi-specific antibody according to any one of items 5 to 7 or the conjugate according to item 11; optionally, the anti-ANG2 heavy-chain single-domain antibody, the recombinant protein or the multi-specific antibody further comprises a detectable label, for example, a radioisotope, a fluorescent substance, a chemiluminescent substance, a colored substance or an enzyme; preferably, the kit is configured to detect the presence or a level of ANG2 in a sample; or
   the kit comprises: (1) the anti-ANG2 heavy-chain single-domain antibody according to any one of items 1 to 3, the recombinant protein according to item 4, the multi-specific antibody according to any one of items 5 to 7 or the conjugate according to item 11, and (2) an antibody or an antigen-binding fragment of the antibody that targets another antigen, and/or a cytotoxic agent, and/or a chemotherapeutic drug, and optionally, an instruction for use.
13. A pharmaceutical composition, comprising the anti-ANG2 heavy-chain single-domain antibody according to any one of items 1 to 3, the recombinant protein according to item 4, the multi-specific antibody according to any one of items 5 to 7 or the conjugate according to item 11, optionally, the pharmaceutical composition further comprises a pharmaceutically acceptable carrier and/or an excipient, preferably, the pharmaceutical composition is in a form suitable for administration through subcutaneous injection, intradermal injection, intravenous injection, intramuscular injection or intralesional injection.
14. Use of the anti-ANG2 heavy-chain single-domain antibody according to any one of items 1 to 3, the recombinant protein according to item 4, the multi-specific antibody according to any one of items 5 to 7 or the conjugate according to item 11 for inhibiting pro-angiogenesis activity of ANG2, preventing and/or treating diseases caused by or related to an angiogenic process such as fundus neovascular disease, rheumatoid arthritis and psoriasis, and/or a tumor, or use for preparing drugs for inhibiting the pro-angiogenesis activity of ANG2, preventing and/or treating the diseases caused by or related to the angiogenic process such as fundus neovascular disease, rheumatoid arthritis and psoriasis, and/or the tumor.

It should be understood that within the scope of the present disclosure, the above technical features of the present disclosure and technical features specifically described below (e.g., examples) can be combined with each other to form a new or preferred technical solution. Details will not be described here for lack of space.

The terms involved in the present disclosure have conventional meanings understood by those skilled in the art. When a term has two or more definitions, the definitions of the terms used herein are used for including all meanings, which is used and/or acceptable in the art.

Those of ordinary skill in the art may understand that a CDR of an antibody is responsible for the binding specificity of the antibody to an antigen. In the case where sequences of heavy chain and light chain variable regions of the antibody are known, there are several methods for determining the CDR of the antibody at present, including Kabat, IMGT, Chothia and AbM numbering systems. However, each application about a definition of a CDR of the antibody or a variant of the antibody will be within the scope of the terms defined and used herein. If the amino acid sequence of the variable region of the antibody is given, those skilled in the art may generally determine particular CDR without relying on any experimental data other than the sequence.

In the present disclosure, a heavy-chain single-domain antibody is also referred to as a VHH domain, a VHH antibody fragment or a VHH antibody, which is a variable domain of an antigen-binding immunoglobulin referred to as a "heavy-chain antibody" (an antibody devoid of a light chain) (Hamers-Casterman C, Atarhouch T, Muyldermans S, Robinson G, Hamers C, Songa EB, BEndahman N, Hamers R.: "Naturally occurring antibodies devoid of light chains", Nature 363, 446-448, 1993). The VHH domain specifically binds to an epitope without another antigen-binding domain. The VHH domain is a small, stable and efficient antigen recognition unit formed by a single immunoglobulin domain.

The heavy-chain single-domain antibody of the present disclosure may be conjugated to a bioactive substance that has a requirement to form a recombinant protein. In some embodiments, the bioactive substance is selected from, for example, a toxin having enzymatic activity or an active fragment (for example, abrin, ricin A, pseudomonas exotoxin or diphtheria toxin) of the toxin, a tumor necrosis factor or an interferon (for example, IFN-γ), a biological response modifier (for example, lymphokines IL-2, IL-2, IL2-6, IL-10 and GM-CSF) and an Fc fragment. The Fc fragment contained in the recombinant protein of the present disclosure may enable the recombinant protein to form a dimer and prolong an *in vivo* half-life of the recombinant protein. In some embodiments, Fc fragments that may be used in the present disclosure may come from different subtypes of immunoglobulins such as IgG (such as an IgG1, IgG2, IgG3 or IgG4 subtype), IgA1, IgA2, IgD, IgE or IgM.

The antibody of the present disclosure may be: (i) a polypeptide where one or more conservative or non-conservative amino acid residues (preferably the conservative amino acid residue) are substituted, or (ii) a polypeptide having a substituent in one or more amino acid residues, or (iii) a polypeptide formed through the fusion of a mature polypeptide with another compound (for example, a compound that lengthens a half-life of the polypeptide, for example, polyethylene glycol), or (iv) a polypeptide (for example, a preamble sequence, or a secretory sequence, or a sequence used for purifying this polypeptide, or a fibrinogen sequence, or a fusion protein formed with a 6His tag) formed through the fusion of an additional amino acid sequence to this polypeptide sequence. According to the teachings herein, these fragments, derivatives and analogs belong to the scope well known to those skilled in the art.

### Conservative substitution

A "conservative amino acid substitution" refers to the substitution of an amino acid residue with an amino acid residue with a similar side chain. Families of amino acid residues with similar side chains have been defined in the art. These families include amino acids with basic side chains (such as lysine, arginine and histidine), amino acids with acidic side chains (such as aspartic acid and glutamic acid), amino acids with uncharged polar side chains (such as glycine, asparagine, glutamine, serine, threonine, tyrosine and cysteine), amino acids with non-polar side chains (such as alanine, valine, leucine, isoleucine, proline, phenylalanine, methionine and tryptophan), amino acids with β-branched side chains (such as threonine, valine and isoleucine) and amino acids with aromatic side chains (such as tyrosine, phenylalanine, tryptophan and histidine). Therefore, a non-essential amino acid residue of an immunoglobulin polypeptide is preferably substituted by another amino acid residue from the same side chain family. In some other embodiments, a string of amino acids may be substituted by a structurally similar amino acid string. The latter differs in an order and/or a composition of a side chain family.

Non-limiting examples of the conservative amino acid substitution are provided in the following table, where a similarity score of 0 or higher indicates a conservative substitution between these two amino acids:

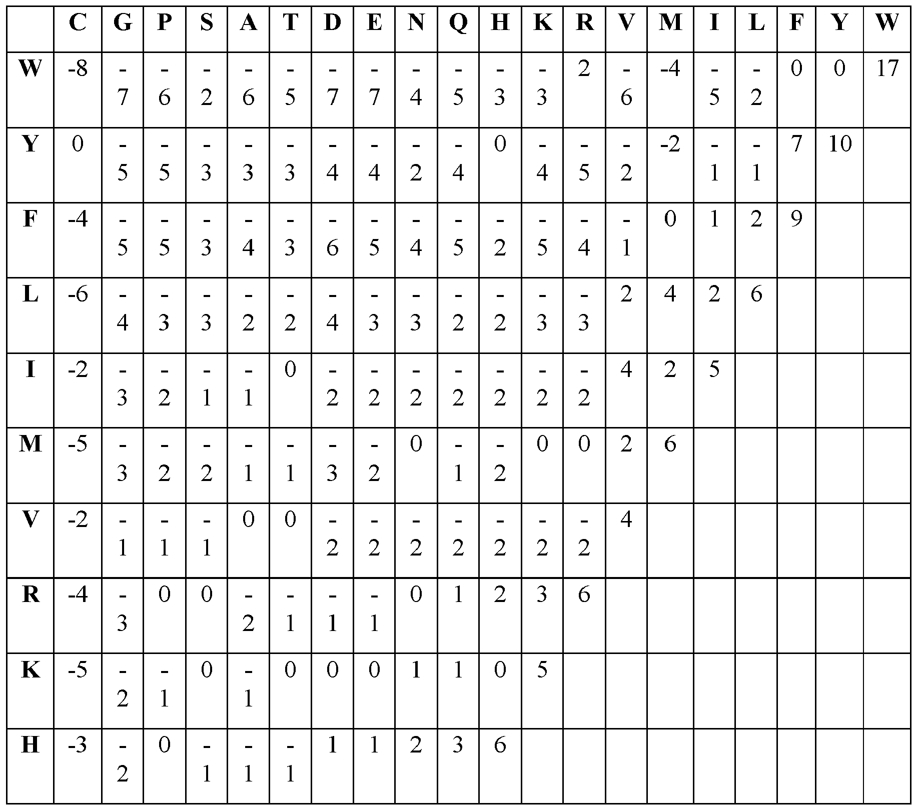

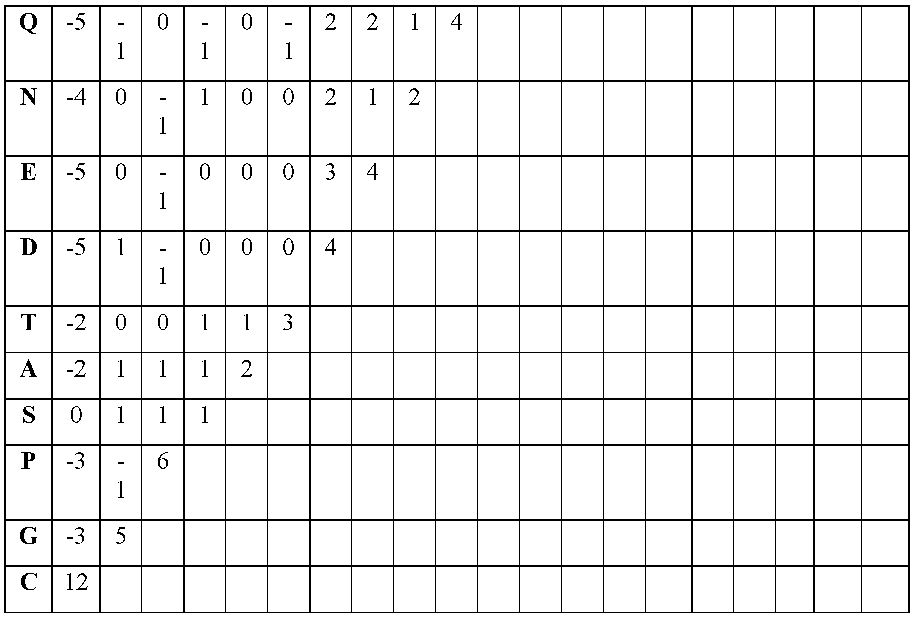

In some embodiments, the conservative substitution is preferably such a substitution, that is, one amino acid in the following groups (a) to (e) is substituted by another amino acid residue in the same group: (a) a small aliphatic, non-polar or slightly polar residue: Ala, Ser, Thr, Pro and Gly; (b) a polar and negatively charged residue and an (uncharged) amide: Asp, Asn, Glu and Gln; (c) a polar and positively charged residue: His, Arg and Lys; (d) a large aliphatic and non-polar residue: Met, Leu, Ile, Val and Cys; and (e) an aromatic residue: Phe, Tyr and Trp.

An especially preferred conservative substitution is as follows: Ala is substituted by Gly or Ser; Arg is substituted by Lys; Asn is substituted by Gln or His; Asp is substituted by Glu; Cys is substituted by Ser; Gln is substituted by Asn; Glu is substituted by Asp; Gly is substituted by Ala or Pro; His is substituted by Asn or Gln; Ile is substituted by Leu or Val; Leu is substituted by Ile or Val; Lys is substituted by Arg, Gln or Glu; Met is substituted by Leu, Tyr or Ile; Phe is substituted by Met, Leu or Tyr; Ser is substituted by Thr; Thr is substituted by Ser; Trp is substituted by Tyr; Tyr is substituted by Trp; and/or Phe is substituted by Val, Ile or Leu.

In some embodiments, the following amino acids have similar structural features and performance:
for example, glycine G, alanine A, valine V, leucine L and isoleucine I all belong to neutral amino acids each containing one amino group and one carboxyl group,
both serine S and threonine T belong to hydroxy-amino acids,
both cysteine C and methionine M belong to sulfur-containing amino acids,
both asparagine N and glutamine Q belong to amide-containing amino acids,
both aspartic acid D and glutamic acid E belong to acidic amino acids,
both lysine K and arginine R belong to basic amino acids,
both phenylalanine F and tyrosine Y belong to aromatic amino acids, and
tryptophan W, histidine H and proline P all belong to heterocyclic amino acids. Therefore, the amino acids have the similar structural features and performance and may be conservatively substituted with each other.

In some embodiments, the antibody of the present disclosure may be combined with a therapeutic agent (for example, chemotherapeutic drugs such as cisplatin and carboplatin), a prodrug, a peptide, a protein, an enzyme, a virus, a lipid, a biological response modifier, an agent or PEG. The antibody of the present disclosure may be linked to or fused to the therapeutic agent, and the therapeutic agent may include detectable labels, such as a radioactive label, an immunomodulator, a hormone, an enzyme, an oligonucleotide, a photoactive therapeutic agent or a diagnostic agent, a cytotoxic agent, an ultrasound enhancing agent, a non-radioactive label, a combination thereof, and other components known in the art; wherein yhe cytotoxic agent may be a drug or a toxin.

### BRIEF DESCRIPTION OF DRAWINGS

FIG. 1 illustrates biological activity in the case where anti-ANG2 heavy-chain single-domain antibodies-Fc bind to human ANG2.
FIG. 2 illustrates biological activity in the case where anti-ANG2 heavy-chain single-domain antibodies-Fc bind to monkey ANG2.
FIG. 3 illustrates biological activity in the case where anti-ANG2 heavy-chain single-domain antibodies-Fc inhibit human ANG2 from binding to receptors hTIE2 of the human ANG2.
FIG. 4 illustrates biological activity in the case where antibodies inhibit human ANG2 from binding to HUVEC.
FIG. 5 illustrates biological activity in the case where antibodies inhibit human ANG1 from binding to HUVEC.
FIG. 6 illustrates that antibodies inhibit the AKT phosphorylation of human ANG2-dependent HUVEC cells.

### DETAILED DESCRIPTION

The present disclosure is described in detail below by using examples. Those of ordinary skill in the art may understand that the following examples are merely used for description. Therefore, the spirit and scope of the present disclosure are defined by the appended claims. Methods used in the following examples are conventional methods unless otherwise specified, and reagents used in the following examples are commercially available reagents unless otherwise specified.

### Example 1 Screening of heavy-chain single-domain antibodies targeting ANG2

A recombinant human ANG2 protein corresponding to an amino acid at position Lys275-Phe496 of human ANG2 is referred to as hANG2 for short hereinafter with a sequence shown in SEQ ID NO: 32. A sequence of a monkey ANG2 protein is shown in SEQ ID NO: 33, and the monkey ANG2 protein is referred to as cynoANG2 for short hereinafter. Specific amino acid sequence information of ANG2 ECD molecules is shown in Table 1.

**Table 1 Sequence information of recombinant human ANG2**

| Species | Sequence | SEQ ID NO: |
|---|---|---|
| Tag | HHHHHHH | 31 |
| human ANG2 (hANG2) | KEEQISFRDCAEVFKSGHTTNGIYTLTFPNSTEEIKAYCDME AGGGGWTIIQRREDGSVDFQRTWKEYKVGFGNPSGEYWL GNEFVSQLTNQQRYVLKIHLKDWEGNEAYSLYEHFYLSSE ELNYRIHLKGLTGTAGKISSISQPGNDFSTKDGDNDKCICKC SQMLTGGWWFDACGPSNLNGMYYPQRQNTNKFNGIKWY YWKGSGYSLKATTMMIRPADF | 32 |
| monkey ANG2 (cynoANG2) | KEEQISFRDCAEVFKSGHTTNGVYTLTLPNSTEEVKAYCD MEAGGGGWTIIQRREDGSVDFQRTWKEYKVGFGNPSGEY WLGNEFVSQLTNQQRYVLKIHLKDWEGNEAYSLYEHFYLS SEELNYRIHLKGLTGTAGKISSISQPGNDFSTKDADNDKCIC KCSQMLTGGWWFDACGPSNLNGMYYPQRQNTNKFNGIK WYYWKGSGYSLKGTTMMIRPADF | 33 |

### 1.1 Construction of phage libraries of heavy-chain single-domain antibodies

Two alpacas were selected for antigen immunization. After four times of immunization with the human ANG2 protein, lymphocytes in 100 mL peripheral blood of the alpaca were extracted, total RNA was extracted with an RNAiso Plus reagent (TAKARA, 9109), and the extracted RNA was reversely transcribed into cDNA by using a PrimeScript II kit (TAKARA, 6210A). Nucleic acid fragments each having a length of 750 bp of heavy chain antibody variable regions and constant regions CH2 were amplified first through a PCR amplification method, and then the nucleic acid fragments each having a length of 750 bp recovered in the previous step were used as templates to amplify target fragments, that is, fragments of the heavy chain antibody variable regions. Vectors pComb3XSS (NBbiolab, NPL-001) and the target fragments were separately subjected to enzyme digestion by using SfiI. The enzyme digestion was performed overnight at 50°C. The target fragments were recovered and ligated according to a ligation molar ratio of vector : fragment = 1 : 3. Ligation products were electrotransformed into *Escherichia coli* competent cells TG1, and ligation products of each alpaca were transformed through electrical shock 10 times. Library volumes were calculated through gradient dilution and plating, and sizes of phage libraries of the two alpacas were 1.21×10⁹ and 1×10⁹, respectively. 96 clones were randomly selected from a titer plate for identification. The results show that an insertion rate is 100%.

### 1.2 Screening of heavy-chain single-domain antibodies targeting ANG2

A plate was coated with the hANG2 protein in 10 µg/well and placed overnight at 4°C. On the next day, after two hours of blocking with 1% BSA at room temperature, 100 µL phages (2×10⁹ pfu/well, from the phage library of the heavy-chain single-domain antibody constructed in 1.1) were added and incubated for 1 h at 37°C. Then, washing was performed with PBST (PBS contained 0.05% Tween 20) five times to wash away unbound phages. Finally, phages specifically binding to hANG2 were eluted with glycine-hydrochloric acid (200 mM) and infected with *Escherichia coli* TG1 growing in a logarithmic phase to generate and purify phages for the next round of screening. After the same screening process was repeated for two rounds, the obtained phages were infected with *Escherichia coli* TG1 and plated, and monoclones were selected from the plate and sequenced. According to the sequence alignment results, a protein sequence of each clone was analyzed, and clones with different CDR1, CDR2 and CDR3 sequences were considered as different antibody strains. Finally, ten strains of different antibodies were obtained in total.

**Table 2 Amino acid sequence information of heavy-chain single-domain antibody variable regions (Numbering Scheme IMGT)**

| Clone No. | Amino Acid Sequence of Antibody Variable Region (CDR is underlined) | SEQ ID NO.: |
|---|---|---|
| B1-G8 | QVQLVESGGGLVQTGDSLRLSCVAPGISFSNRGMAWYRQ APGKQRELVAAIDSAGSQRYAGSVKDRFTISRDNAKNTVF LQMNSLKPEDTAVYYCNQPRFNLWGQGTQVTVSS CDR1: **GISFSNRG** (SEQ ID NO: 21) | 1 |
| | CDR2: **IDSAGSQ** (SEQ ID NO: 22) | |
| | CDR3: **NQPRFNL** (SEQ ID NO: 23) | |
| B1-G3 | EVQVVESGGGLVQPGGSLRLSCVASMPSGISFSNRGMAW YRQAPGKQRELVAAIDSAGSTRYQDSVKGRFTISRDNAKN TVFLQMNSLKPEDTAVYYCNQPAFKAWGQGTQVTVSS CDR1: **MPSGISFSNRG** (SEQ ID NO: 34) | 2 |
| | CDR2: **IDSAGST** (SEQ ID NO: 35) | |
| | CDR3: **NQPAFKA** (SEQ ID NO: 36) | |
| B4-D3 | QLQLVESGGGLVQPGGSLRLSCVASGASGISFSNRGMAW YRQAPGKQRELVAAIDSAGSPAYAGSVKGRFTISRDNAKN TVFLQMNSLKPEDTAVYYCNQPRFNLWGLGTQVTVSS CDR1: **GASGISFSNRG** (SEQ ID NO: 37) | 3 |
| | CDR2: **IDSAGSP** (SEQ ID NO: 38) | |
| | CDR3: **NQPRFNLWG** (SEQ ID NO: 39) | |
| B2-E2 | EVQLVESGGGLVQPGESLRLSCVASGFPSGFTFSDYQMY WYRQAPGKERELVAIISNDGLRKTYADSVKGRFTISRDNA KNTMFLQMNSLKPEDTAVYYCNVRRANRDDWGQGTQV TVSS CDR1: **GFPSGFTFSDYQ** (SEQ ID NO: 40) | 4 |
| | CDR2: **ISNDGLRK** (SEQ ID NO: 41) | |
| | CDR3: **NVRRANRDD** (SEQ ID NO: 42) | |
| B2-F10 | QVQLVESGGGLVQAGGSLRLSCGASGSAVSITLMAWYRQ APGKQRELVAGISNIGDTNYVDSVKDRFTISRINAKNAMY LQMNSLKPEDTAVYYCFAQDWGGHHYWGQGTQVTVSS CDR1: **GSAVSITL** (SEQ ID NO: 43) | 5 |
| | CDR2: **ISNIGDT** (SEQ ID NO: 44) | |
| | CDR3: **FAQDWGGHHY** (SEQ ID NO: 45) | |
| B4-C11 | QVQLVESGGGLVQAGGSLRLSCAASGSTFSNVVAGWYRQ APGKQRELVAGISNIGDTRYADSVKGRFTISRDNAKKAVY LQMNSLKPEDTAVYYCYAKYWARDYWGQGTQVTVSS CDR1: **GSTFSNVVA** (SEQ ID NO: 46) | 6 |
| | CDR2: **ISNIGDT** (SEQ ID NO: 44) | |
| | CDR3: **YAKYWARDY** (SEQ ID NO: 48) | |
| B1-H9 | EVQLVESGGDLVQAGGSLRLSCAASGSTFSSTIAGWYRQ APGKQRELVAGISNIGDTRYADSVKGRFTISRDNAKKTVY LQMNSLKPEDTAVYYCYAKYWARDYWGQGTQVTVSS CDR1: **GSTFSSTI** (SEQ ID NO: 49) | 7 |
| | CDR2: **ISNIGDT** (SEQ ID NO: 44) | |
| | CDR3: **YAKYWARDY** (SEQ ID NO: 48) | |
| B3-H7 | EVQVVESGGGLVQAGGSLRLSCAASGSTAINSVAGWYRQ APGKQRELVAGISNIGDTRYADSVKGRFTISRDNAKKTVY LQMNSLKSEDTAVYYCYAKYWARDYWGQGTQVTVSS CDR1: **GSTAINSV** (SEQ ID NO: 52) | 8 |
| | CDR2: **ISNIGDT** (SEQ ID NO: 44) | |
| | CDR3: **YAKYWARDY** (SEQ ID NO: 48) | |
| B3-C11 | EVQLVESGGGLVQAGGSLRLSCFVSGSTFSASLMGWYRQ APGKERELVAGISNIGNTVYADSAKGRFTISRDGAKNTVH LQMNALKPEDTAVYYCYATVPRAAWGQGTQVTVSS CDR1: **GSTFSASL** (SEQ ID NO: 55) | 9 |
| | CDR2: **ISNIGNT** (SEQ ID NO: 56) | |
| | CDR3: **YATVPRAA** (SEQ ID NO: 57) | |
| B2-A6 | QLQLVESGGGLVQAGGSLRLSCFVSGSTFSASLMGWYRQ APGKERELVAGISNIGNTVYADSAKGRFTISRDGAKNTVH LQMNALKPEDTAVYYCYATVPRAAWGQGTQVTVSS CDR1: **GSTFSASL** (SEQ ID NO: 55) | 10 |
| | CDR2: **ISNIGNT** (SEQ ID NO: 56) | |
| | CDR3: **YATVPRAA** (SEQ ID NO: 57) | |

### Example 2 Preliminary evaluation and identification of heavy-chain single-domain antibodies targeting ANG2

### 1. Expression of heavy-chain single-domain antibodies in host strains Escherichia coli

Single colonies of TG1 host strains of the obtained ten strains of heavy-chain single-domain antibodies were selected, inoculated and cultured overnight. On the next day, the overnight strains were transferred, amplified, induced by 0.5 mM IPTG and cultured in a shaker overnight at 37°C. On the next day, centrifugation was performed to collect supernatant for detection.

### 2. Detection of affinities of ten heavy-chain single-domain antibodies through ELISA

An ELISA plate was separately coated with an hANG2 protein solution and BSA in 2 µL/well and placed overnight at 4°C. Supernatant was discarded, and 300 µL blocking solution (PBS containing 3% BSA) was added to each well and blocked for 2 h at 37°C. Supernatant collected after centrifugation was added to wells of the ELISA plate in 200 µL per well and subjected to standing incubation for 2 h at room temperature, and supernatant was discarded. Washing was performed with PBST (PBS contained 0.1% Tween 20) in 200 µL/well three times. Diluted HRP-labeled anti-VHH secondary antibodies (Genscript, A01861) were added in a volume of 100 µL/well and incubated for 1 h at room temperature, wherein the secondary antibodies were used after dilution according to 1:10000 with a diluent of PBS containing 1% BSA. Washing was performed with PBST in 200 µL/well five times. A TMB color developing solution (BD, 55214) was added in 100 µL/well, and color developing was performed for 8 min at 37°C.2M HCl stopping solution was added in 100 µL/well. After the stopping solution was added, values at 450 nm were read with a microplate reader within 30 minutes. The results are shown in the following Table 3:

**Table 3 OD values obtained after heavy-chain single-domain antibodies bind to hANG2 antigens**

| Clone No. | OD Value Obtained after Binding to hANG2 | OD Value Obtained after Binding to BSA |
|---|---|---|
| B1-G8 | 2.021 | 0.065 |
| B1-G3 | 1.572 | 0.067 |
| B4-D3 | 1.679 | 0.059 |
| B2-E2 | 1.962 | 0.070 |
| B2-F10 | 2.061 | 0.063 |
| B4-C11 | 1.58 | 0.071 |
| B1-H9 | 1.495 | 0.058 |
| B3-H7 | 1.82 | 0.056 |
| B3-C11 | 1.63 | 0.071 |
| B2-A6 | 2.39 | 0.049 |

### Example 3 Preparation of plasmids of anti-ANG2 heavy-chain single-domain antibodies-Fc

Coding DNA sequences corresponding to monoclonal antibody heavy chain variable regions (SEQ ID NOs: 1 to 10) cloned through PCR were ligated to coding DNA corresponding to a human IgG1 heavy chain constant region (GenBank No. AK303185.1) to construct heavy-chain antibodies to obtain the expression plasmids of the anti-ANG2 heavy-chain single-domain antibodies-Fc, and vectors were generally pcDNA3.1(-) (purchased from Invitrogen) or other eukaryotic expression vectors. A variable region and a constant region of a heavy chain antibody protein sequence were linked by a linker peptide, and the linker peptide was (GGGGS)n, where n = 1, 2 or 3.

**Table 4 Amino acid sequences of anti-ANG2 heavy-chain single-domain antibodies-Fc**

| Clone No. | Amino Acid Sequence of Antibody | SEQ ID NO |
|---|---|---|
| B1-G8-Fc | QVQLVESGGGLVQTGDSLRLSCVAPGISFSNRGMAWYRQ APGKQRELVAAIDSAGSQRYAGSVKDRFTISRDNAKNTVF LQMNSLKPEDTAVYYCNQPRFNLWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST | 11 |
| | YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | |
| B1-G3-Fc | EVQVVESGGGLVQPGGSLRLSCVASMPSGISFSNRGMAW YRQAPGKQRELVAAIDSAGSTRYQDSVKGRFTISRDNAKN TVFLQMNSLKPEDTAVYYCNQPAFKAWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 12 |
| B4-D3-Fc | QLQLVESGGGLVQPGGSLRLSCVASGASGISFSNRGMAWY RQAPGKQRELVAAIDSAGSPAYAGSVKGRFTISRDNAKNT VFLQMNSLKPEDTAVYYCNQPRFNLWGLGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 13 |
| B2-E2-Fc | EVQLVESGGGLVQPGESLRLSCVASGFPSGFTFSDYQMYW YRQAPGKERELVAIISNDGLRKTYADSVKGRFTISRDNAK NTMFLQMNSLKPEDTAVYVCNVRRANRDDWGQGTQVT VSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 14 |
| B2-F10-Fc | QVQLVESGGGLVQAGGSLRLSCGASGSAVSITLMAWYRQ APGKQRELVAGISNIGDTNYVDSVKDRFTISRINAKNAMY LQMNSLKPEDTAVYYCFAQDWGGHHYWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 15 |
| B4-C11-Fc | QVQLVESGGGLVQAGGSLRLSCAASGSTFSNVVAGWYRQ APGKQRELVAGISNIGDTRYADSVKGRFTISRDNAKKAVY LQMNSLKPEDTAVYYCYAKYWARDYWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST | 16 |
| | YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | |
| B1-H9-Fc | EVQLVESGGDLVQAGGSLRLSCAASGSTFSSTIAGWYRQA PGKQRELVAGISNIGDTRYADSVKGRFTISRDNAKKTVYL QMNSLKPEDTAVYYCYAKYWARDYWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 17 |
| B3-H7-Fc | EVQVVESGGGLVQAGGSLRLSCAASGSTAINSVAGWYRQ APGKQRELVAGISNIGDTRYADSVKGRFTISRDNAKKTVY LQMNSLKSEDTAVYYCYAKYWARDYWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 18 |
| B3-C11-Fc | EVQLVESGGGLVQAGGSLRLSCFVSGSTFSASLMGWYRQ APGKERELVAGISNIGNTVYADSAKGRFTISRDGAKNTVH LQMNALKPEDTAVYYCYATVPRAAWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 19 |
| B2-A6-Fc | QLQLVESGGGLVQAGGSLRLSCFVSGSTFSASLMGWYRQ APGKERELVAGISNIGNTVYADSAKGRFTISRDGAKNTVH LQMNALKPEDTAVYYCYATVPRAAWGQGTQVTVSS GGGGSGGGGSGGGGS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK | 20 |

### Example 4 Expression and purification of anti-ANG2 heavy-chain single-domain antibodies-Fc

Plasmid extraction was performed by using an Endofree Plasmid Maxi Kit (Qiagen, Cat. No. 12391), and specific operations were performed according to an instruction provided by a manufacturer. According to the instruction provided by the manufacturer, CHO-S cells were cultured in a CD CHO medium (Gibco, Cat. No. 10743-029) in a 5% CO₂ cell incubator at 37°C. After the cells were prepared, the plasmids containing the sequences of the anti-ANG2 heavy-chain single-domain antibodies-Fc were co-transfected into the CHO-S cells to express the antibodies-Fc. On the next day after the transfection, the culture temperature was reduced to 32°C, and 3.5% 2×EFC+ (Gibco, Cat. No. A2503105) was added every day. After 14 days of culture, expression supernatant was obtained after 800×g centrifugation. The expression supernatant was filtered with a 0.22 µm filter membrane. Antibodies-Fc in culture supernatant were obtained through protein A affinity chromatography and cation-exchange chromatography purification. A concentration of the purified antibodies-Fc was determined by a UV absorbance at 280 nm and an extinction coefficient corresponding to each protein. The purity and homogeneity of the antibodies-Fc were assessed through SDS-PAGE and SE-HPLC. Alternatively, secondary purification was performed through ion exchange and SEC of Superdex 200 to prepare high-purity antibody-Fc samples for later use.

### Example 5 Affinities of binding anti-ANG2 heavy-chain single-domain antibodies-Fc to ANG2

In this example, the affinities of the ten anti-ANG2 heavy-chain single-domain antibodies-Fc were evaluated through the ELISA detection method.

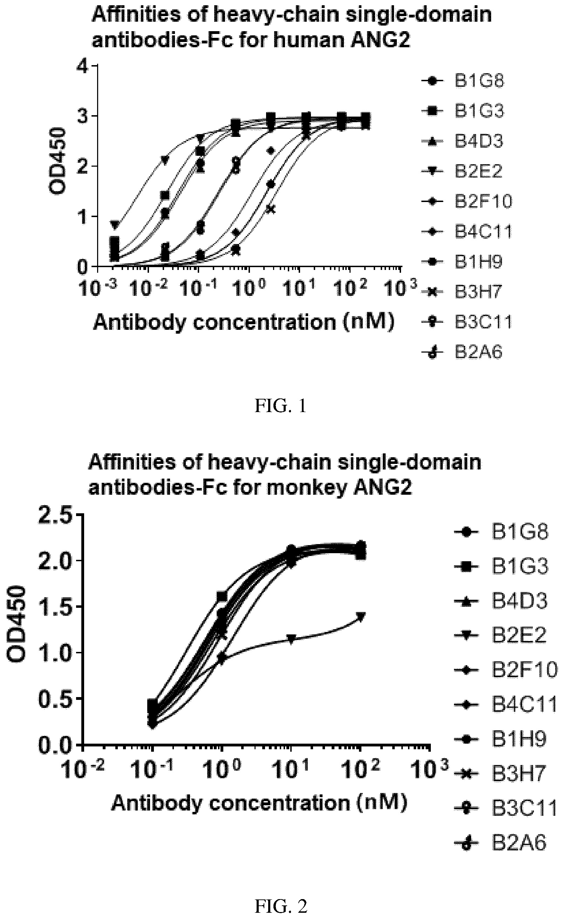

An ELISA plate was coated with an ANG2 protein solution in 2 µL/well and placed overnight at 4°C. Supernatant was discarded, and 300 µL blocking solution (PBS containing 3% BSA) was added to each well and blocked for 2 h at 37°C. The anti-ANG2 heavy-chain single-domain antibodies-Fc were subjected to gradient dilution with a diluent of PBS containing 1% BSA. For example, an initial concentration of dilution was 500 nM, and the anti-ANG2 heavy-chain single-domain antibodies-Fc were diluted 10 times to 8 concentration gradients. The diluted anti-ANG2 heavy-chain single-domain antibodies-Fc were added to wells of the ELISA plate in 200 µL per well and subjected to standing incubation for 2 h at room temperature, and supernatant was discarded. Washing was performed with PBST (PBS contained 0.1% Tween 20) in 200 µL/well three times. Diluted HRP-labeled anti-human Fc secondary antibodies (SIGMA, A8667) were added in a volume of 100 µL/well and incubated for 1 h at room temperature, wherein the secondary antibodies were used after dilution according to 1:20000 with a diluent of PBS containing 1% BSA. Washing was performed with PBST in 200 µL/well five times. A TMB color developing solution (BD, 55214) was added in 100 µL/well, and color developing was performed for 8 min at 37°C. 2M HCl stopping solution was added in 100 µL/well. After the stopping solution was added, values at 450 nm were read with the microplate reader within 30 minutes. The data were analyzed by using GraphPad Prism6.0 software, and the fitting of the affinities was performed to obtain EC50 values. The results are shown in FIG. 1, FIG. 2 and Table 5. B2-E2-Fc has the highest affinities for the human ANG2 and monkey ANG2 proteins.

**Table 5 Affinities of heavy-chain single-domain antibodies for ANG2 proteins**

| Antibody-Fc | Anti-hANG2 EC50 nM | Anti-cynoANG2 EC50 nM |
|---|---|---|
| B1-G8-Fc | 0.03923 | 0.5893 |
| B1-G3-Fc | 0.02332 | 0.3065 |
| B4-D3-Fc | 0.04241 | 0.7072 |
| B2-E2-Fc | 0.005842 | 0.2727 |
| B2-F10-Fc | 1.112 | 1.44 |
| B4-C11-Fc | 2.275 | 0.5418 |
| B1-H9-Fc | 2.235 | 0.6734 |
| B3-H7-Fc | 3.507 | 0.9301 |
| B3-C11-Fc | 0.2415 | 0.624 |
| B2-A6-Fc | 0.2617 | 0.6409 |

### Example 6 Anti-ANG2 heavy-chain single-domain antibodies-Fc inhibit hANG2 from binding to receptors hTIE2 of hANG2

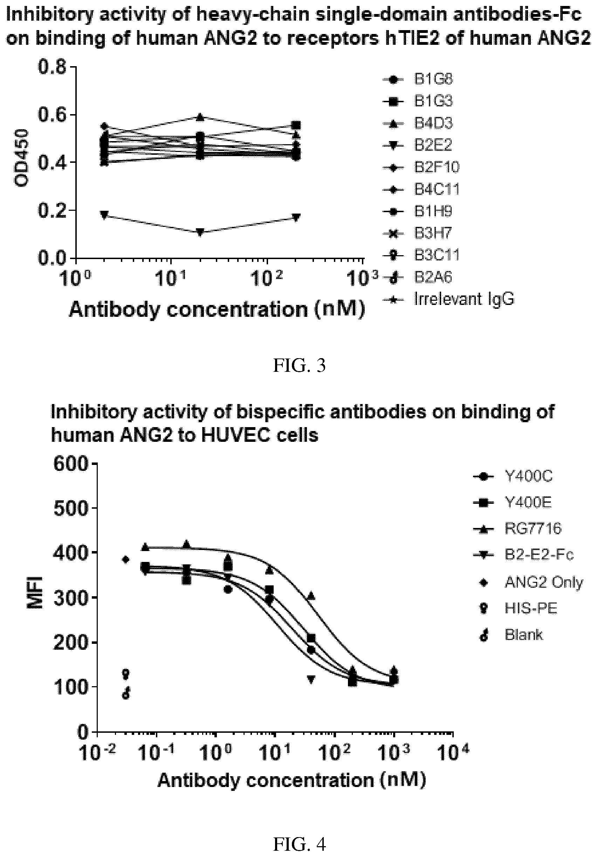

A 96-well plate was treated with 100 µL and 2 µg/mL hTIE2-Fc (Novoprotein, CW98) prepared in PBS and incubated overnight at 4°C. Then, the plate was washed with a PBS wash buffer containing 0.1% Tween-20 three times, and 300 µL PBS containing 1% BSA was added and blocked for 2 h. The anti-ANG2 heavy-chain single-domain antibodies-Fc to be detected were diluted and mixed with hANG2 having a final concentration of 10 µg/mL. After one hour of incubation at 37°C, the antigen-antibody mixture was added to the 96-well plate and incubated for 1 h at 37°C. After washing was performed with a PBS wash buffer containing 0.1% Tween-20, diluted HRP-labeled anti-His secondary antibodies (Proteintech, 66005-1-Ig) were added in a volume of 100 µL/well and incubated for 1 h at room temperature, wherein the secondary antibodies were used after dilution according to 1:2000 with a diluent of PBS containing 1% BSA. Washing was performed with PBST in 200 µL/well five times. A TMB color developing solution (BD, 55214) was added in 100 µL/well, and color developing was performed for 8 min at 37°C. 2M HCl stopping solution was added in 100 µL/well. After the stopping solution was added, values at 450 nm were read with the microplate reader within 30 minutes. The data were analyzed by using the GraphPad Prism6.0 software, and the fitting of affinities was performed to obtain EC50 values. The results are shown in FIG. 3. B2E2-Fc has an apparent inhibitory effect on the binding of the human ANG2 to the receptors TIE2 of the human ANG2.

### Example 7 Evaluation of acid stability and thermal stability of anti-ANG2 heavy-chain single-domain antibody-Fc

B2-E2-Fc was evaluated according to the following acid stability and thermal stability evaluation methods. When anti-ANG2 heavy-chain single-domain antibody-Fc molecules were subjected to protein A affinity chromatography, in a step of acid elution (a pH = 3.5 citric acid buffer was used), a solution of the eluted anti-ANG2 heavy-chain single-domain antibody-Fc was not neutralized. After the anti-ANG2 heavy-chain single-domain antibody-Fc was maintained in the buffer for a period of time, a sample was taken at 30 min, 1/10 volume 1 M Tris-HCl (pH = 8.0) was added to the sample for neutralization, and the sample was subjected to HPLC-SEC detection. After the antibody molecules were treated for 30 min at pH = 3.5, no aggregation or degradation phenomenon occurred, and a change in purity was less than 4%, indicating that the antibody can remain stable in an acidic environment. Moreover, after 14 days of incubation in an incubator at 40°C, the sample was subjected to HPLC-SEC detection. No aggregation or degradation phenomenon occurred, and a change in purity was less than 4%, indicating that the antibody can remain stable in a 40°C environment. The results in Table 6 and Table 7 show that B2-E2-Fc has good acid stability and thermal stability.

**Table 6 Evaluation results of acid stability of anti-ANG2 heavy-chain single-domain antibody-Fc**

| Sample | pH = 3.5, 30 min | |
|---|---|---|
| | Purity Detected through SEC before Treatment | Purity Detected through SEC after Treatment |
| B2-E2-Fc | 96.78% | 95.59% |

**Table 7 Evaluation results of thermal stability of anti-ANG2 heavy-chain single-domain antibody-Fc**

| Sample | Detection of thermal stability, treatment at 40°C | | |
|---|---|---|---|
| | Purity Detected through SEC on Day 0 | Purity Detected through SEC on Day 7 | Purity Detected through SEC on Day 14 |
| B2-E2-Fc | 96.78% | 95.99% | 94.74% |

### Example 8 Construction of bispecific antibodies and applications

### (1) Construction of bispecific antibodies

Reverse translation of DNA coding and synthesis of DNA fragments were performed according to the amino acid sequences of the bispecific antibodies shown in Table 8, and constructed into expression vectors pcDNA3.1 and transiently transfected into 293 or CHO cells for expression. Supernatant was obtained and subjected to protein purification to obtain bispecific antibodies Y400C and Y400E each having purity of no less than 95%. Reference may be made to each of the preceding examples for vector construction, transient transfection and protein purification methods in detail. A structure of the bispecific antibody includes a light chain and a heavy chain, where the light chain and the heavy chain are paired to form an interchain disulfide bond, and two heavy chains are paired to form an interchain disulfide bond, wherein the heavy chain is (VH)-(CH1)-(hinge region)-(Fc)-(linker peptide)-(VHH), and the light chain is (VL)-(light chain constant region), wherein VH and VL are heavy chain and light chain variable regions of an anti-VEGF antibody, and VHH is a variable region of an anti-ANG2 heavy-chain single-domain antibody.

**Table 8 Amino acid sequences of bispecific antibodies (Numbering scheme IMGT)**

| **Bispecific Antibody** | **Chain** | **Domain** | **Amino Acid Sequence (CDR is underlined, and mutation site is in block)** | **SEQ ID NO** |
|---|---|---|---|---|
| Y400C | heavy chain | anti-VEGF heavy chain variable region (VH) | | 24 |
| | | | HCDR1: **GYDFTHYG** (SEQ ID NO: 50) | |
| | | | HCDR2: **INTYTGEP** (SEQ ID NO: 51) | |
| | | | HCDR3: **AKYPYYYGTSHWYFDV**(SEQ ID NO: 53) | |
| | | CH1 | ASTKGPSVFPLAPSSKSTSGGTAALGCLVK DYFPEPVTVSWNSGALTSGVHTFPAVLQS SGLYSLSSVVTVPSSSLGTQTYICNVNHKP SNTKVDKKVEPKSC | 25 |
| | | hinge region | DKTHTCPPCP | 26 |
| | | Fc constant region | APELLGGPSVFLFPPKPKDTLMISRTPEVT CVVVDVSHEDPEVKFNWYVDGVEVHNA KTKPREEQYNSTYRVVSVLTVLHQDWLN GKEYKCKVSNKALPAPIEKTISKAKGQPR EPQVYTLPPSRDELTKNQVSLTCLVKGFYP SDIAVEWESNGQPENNYKTTPPVLDSDGS FFLYSKLTVDKSRWQQGNVFSCSVMHEA LHNHYTQKSLSLSPG | 29 |
| | | linker peptide | GGGGS | 30 |
| | | anti-ANG2 heavy-chain single-domain antibody (VHH) | EVQLVESGGGLVQPGESLRLSCVASGFPS GFTFSDYQMYWYRQAPGKERELVAIISN DGLRKTYADSVKGRFTISRDNAKNTMFL QMNSLKPEDTAVYYCNVRRANRDDWGQ GTQVTVSS | 4 |
| | light chain | anti-VEGF light chain variable region (VL) | | 27 |
| | | | LCDR1: **QDISNY** (SEQ ID NO: 54) | |
| | | | LCDR2: **FTS** (SEQ ID NO: 58) | |
| | | | LCDR3: **QQYSTVPWT** (SEQ ID NO: 59) | |
| | | light chain constant region | | 28 |
| Y400E | heavy chain | anti-VEGF heavy chain variable region | | 24 |
| | | CH1 | ASTKGPSVFPLAPSSKSTSGGTAALGCLVK DYFPEPVTVSWNSGALTSGVHTFPAVLQS SGLYSLSSVVTVPSSSLGTQTYICNVNHKP SNTKVDKKVEPKSC | 25 |
| | | hinge region | DKTHTCPPCP | 26 |
| | | Fc constant region | APELLGGPSVFLFPPKPKDTLMISRTPEVT CVVVDVSHEDPEVKFNWYVDGVEVHNA KTKPREEQYNSTYRVVSVLTVLHQDWLN GKEYKCKVSNKALPAPIEKTISKAKGQPR EPQVYTLPPSRDELTKNQVSLTCLVKGFYP SDIAVEWESNGQPENNYKTTPPVLDSDGS FFLYSKLTVDKSRWQQGNVFSCSVMHEA LHNHYTQKSLSLSPG | 29 |
| | | linker peptide | GGGGSGGGGSGGGGS | 47 |
| | | anti-ANG2 heavy-chain single-domain antibody | EVQLVESGGGLVQPGESLRLSCVASGFPS GFTFSDYQMYWYRQAPGKERELVAIISN DGLRKTYADSVKGRFTISRDNAKNTMFL QMNSLKPEDTAVYYCNVRRANRDDWGQ GTQVTVSS | 4 |
| | light chain | anti-VEGF light chain variable region | | 27 |
| | | light chain constant region | | 28 |

### (2) Investigation of thermal stability of bispecific antibodies

After the bispecific antibodies were placed in an incubator for 14 days or 28 days of treatment at 40°C, the samples were subjected to HPLC-SEC detection. It is found that after the antibody molecules of the present disclosure were treated for 28 days at 40°C, no aggregation or degradation phenomenon occurred, and changes in purity were less than 4%, indicating that the antibodies can remain stable in a 40°C environment, as shown in Table 9.

**Table 9 Evaluation results of thermal stability of antibodies**

| Sample | Detection of thermal stability, treatment at 40°C | | |
|---|---|---|---|
| | Purity Detected through SEC on Day 0 | Purity Detected through SEC on Day 14 | Purity Detected through SEC on Day 28 |
| Y400C | 98.24% | 97.20% | 99.10% |
| Y400E | 97.90% | 96.93% | 99.27% |

The results show that the diabody molecules Y400C and Y400E both have good thermal stability.

### (3) Binding of bispecific antibodies to hANG2

The test antibodies were captured by using a Sensor Chip ProteinA chip (GE, Cat. No. 28995056), and the antigen human ANG2 protein (623-AN-025/CF, R&D) was used as an analyte to detect kinetics and affinity data obtained after the antigen human ANG2 protein bound to the test samples. An initial concentration of the detection of the binding of the antigen to the test samples was 10 nM. On this basis, the antigen was subjected to 2-fold gradient dilution, that is, concentrations of the diluted antigen were 10 nM, 5 nM, 2.5 nM, 1.25 nM and 0.625 nM, respectively. The antigen was injected from the low concentration to the high concentration in sequence. One negative control (1×HBS-EP+buffer) and one repeated concentration (generally, the lowest concentration was repeated) were set. Before the injection, a flushing process of Start up (1×HBS-EP + buffer) was performed at least three times to balance the system. Binding and dissociation trends of the antigen and the test samples were detected. After the dissociation was completed, a regenerating agent was injected to regenerate the chip. After the chip regeneration was completed, the next concentration was detected. After the detection was completed, a data fitting experiment was performed through a 1:1 Binding fitting manner in data analysis software (Biacore T200 Evaluation Software). The results are shown in Table 10.

**Table 10 Detection results of affinities of antibodies for human ANG2**

| Sample | Antigen | ka(1/Ms) | kd(1/s) | KD(M) |
|---|---|---|---|---|
| Y400C | hANG2 | 1.142E+06 | 6.224E-04 | 5.450E-10 |
| Y400E | | 1.928E+06 | 8.731E-04 | 4.529E-10 |

The detection results of the affinities for the human ANG2 show that the bispecific antibodies have relatively strong affinities for the human ANG2.

### (4) Bispecific antibodies inhibit hANG2 from binding to HUVEC cells

HUVEC cells (H-003, Allcells) in a good growth state were prepared into a single cell suspension. After cell counting, the cells were plated in a 96-well plate in 10⁵ cells/well. A final concentration of the human ANG2 protein (623-AN-025/CF, R&D) was 10 µg/mL, which was constant. The antibodies to be detected were added to each well according to an experimental design, respectively. The highest concentration of the antibodies was 1000 nM, the antibodies were subjected to 5-fold dilution in sequence and 7 concentration gradients were set in total, and meanwhile, a blank control and a positive control RG7716 (ATAD00534, Atagenix) were set. After the cells were incubated for 30 min at room temperature, cells in each well were washed three times and resuspended, and fluorescence-labeled secondary antibodies PE anti-His (362603, Biolegend) were added and incubated in darkness for 30 min at room temperature. The cells in each well were washed three times, resuspended and loaded on a flow cytometer for detection. As shown in FIG. 4, blocking activity on ANG2 and the HUVEC cells of the bispecific antibodies Y400C and Y400E is better than that of the control positive antibody.

**Table 11 Biological activity in the case where antibodies inhibit human ANG2 from binding to HUVEC**

| Sample | IC50 (nM) |
|---|---|
| Y400C | 19.43 |
| Y400E | 30.29 |
| B2-E2-Fc | 11.19 |
| RG7716 | 53.87 |

### (5) Bispecific antibodies did not inhibit hANG1 from binding to HUVEC cells

Among biological functions related to this signal pathway ANG1/ANG2-TIE2, preserving ANG1 activity is beneficial to certain treatments of anti-angiogenesis. Therefore, the antibodies of the present disclosure specifically bind to ANG2 but do not bind to ANG1, and meanwhile, the antibodies of the present disclosure only inhibit ANG2 from binding to receptors TIE2 of ANG2 but do not inhibit ANG1 from binding to receptors TIE2 of ANG1. The antibodies of the present disclosure are particularly useful in inhibiting pro-angiogenesis activity of ANG2 and treating diseases and conditions caused by or related to an angiogenic process.

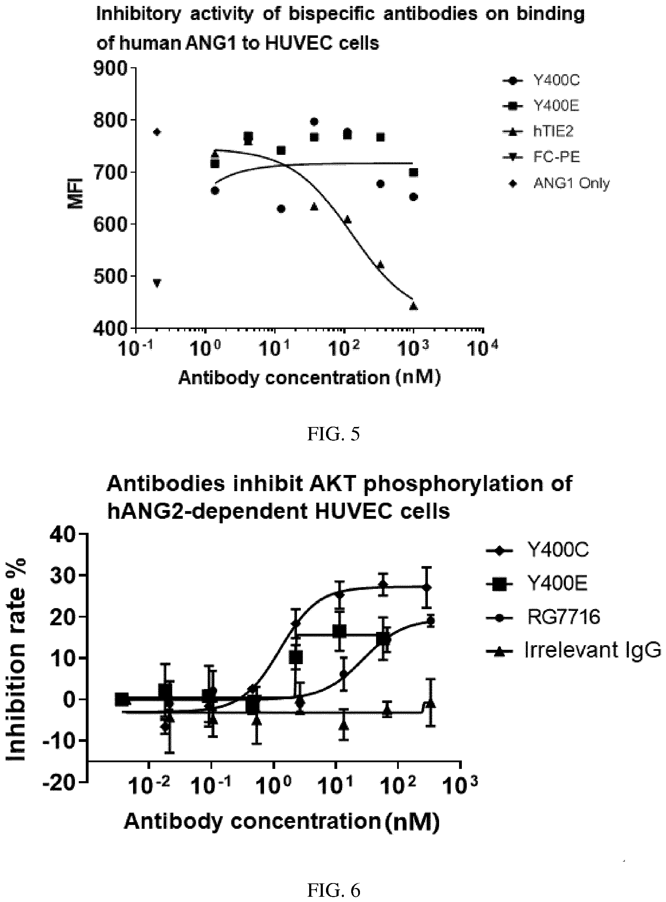

HUVEC cells (H-003, Allcells) in a good growth state were prepared into a single cell suspension. After cell counting, the cells were plated in a 96-well plate in 10⁵ cells/well. A final concentration of the human ANG1 protein (623-AN/CF, R&D) was 10 µg/mL, which was constant. The antibodies to be detected were added to each well according to an experimental design, respectively. The highest concentration of the antibodies was 1000 nM, the antibodies were subjected to 5-fold dilution in sequence and 7 concentration gradients were set in total, and meanwhile, a blank control and a positive control hTIE2-Fc (Novoprotein, CW98) were set. After the cells were incubated for 30 min at room temperature, cells in each well were washed three times and resuspended, and fluorescence-labeled secondary antibodies PE anti-His (362603, Biolegend) were added and incubated in darkness for 30 min at room temperature. The cells in each well were washed three times, resuspended and loaded on the flow cytometer for detection. As shown in FIG. 5, the bispecific antibodies Y400C and Y400E have no binding blocking activity on ANG1 and the HUVEC cells.

### (6) Bispecific antibodies inhibit AKT phosphorylation of hANG2-dependent HUVEC cells

The receptors of the ANG2 protein are naturally expressed on surfaces of the HUVEC cells. When ANG2 binds to the receptors on the surfaces of the cells, AKT phosphorylation reactions are activated. A Phospho-AKT analysis kit (64AKSPE1-1, Cisbio) includes two labeled antibodies: one with a donor fluorophore and the other with a receptor. A reason for selecting the first antibody is that the first antibody specifically binds to a phosphorylation motif on the protein, and a reason for selecting the second antibody is that an ability of the second antibody to identify the protein is independent of a phosphorylation state of the protein. The phosphorylation of the protein enables the formation of an immune complex to relate to the labeled antibodies and enables the donor fluorophore to be in close proximity to the receptor, thereby generating a signal. In this experiment, HUVEC cells were co-incubated with serially diluted samples and ANG2 with a constant concentration. Functional activity of ANG2 and an inhibitory effect of the samples on the functional activity of ANG2 were evaluated according to a degree of AKT phosphorylation of the HUVEC cells. HUVEC cells (70013502, ATCC) in a good growth state were prepared into a single cell suspension. After cell counting, the cells were plated in a 96-well plate in 10⁵ cells/well. A final concentration of the human ANG2 protein (623-AN-025/CF, R&D) was 10 µg/mL, which was constant. The antibodies to be detected were added to each well according to an experimental design, respectively. The highest concentration of the antibodies was 1000 nM, the antibodies were subjected to 5-fold dilution in sequence and 8 concentration gradients were set in total, and meanwhile, a blank control and a positive control RG7716 (ATAD00534, Atagenix) were set. After the cells were incubated for 10 min at 37°C, supernatant was removed from wells. After a lysis buffer was added, incubation was performed with shaking for 30 min at room temperature. Cell lysates were pipetted to a 384-well plate, and a detection reagent was added, incubated in darkness for 4 h and detected by using PHERAstar. As shown in FIG. 6, inhibitory activity on the AKT phosphorylation of the hANG2-dependent HUVEC cells of the bispecific antibodies Y400C and Y400E is better than that of the control positive antibody.

**Table 12 Antibodies inhibit AKT phosphorylation of human ANG2-dependent HUVEC cells**

| Sample | IC50 (nM) |
|---|---|
| Y400C | 1.262 |
| Y400E | 2.269 |
| RG7716 | 27.31 |

All the publications mentioned in the present disclosure are incorporated herein by reference as if each publication is separately incorporated herein by reference. In addition, it should be understood that those skilled in the art who have read the disclosure can make various changes or modifications to the present disclosure, and these equivalent forms fall within the scope of the appended claims.

## Claims

1. An anti-ANG2 heavy-chain single-domain antibody, comprising HCDR1, HCDR2 and HCDR3 contained in a heavy chain variable region shown in any one of SEQ ID NOs: 1 to 10 or variants of HCDR1, HCDR2 and HCDR3,
preferably, according to an IMGT numbering system, comprising HCDR1, HCDR2 and HCDR3 selected from a group consisting of:
(1) HCDR1 shown in SEQ ID NO: 21, HCDR2 shown in SEQ ID NO: 22 and HCDR3 shown in SEQ ID NO: 23,
(2) HCDR1 shown in SEQ ID NO: 34, HCDR2 shown in SEQ ID NO: 35 and HCDR3 shown in SEQ ID NO: 36,
(3) HCDR1 shown in SEQ ID NO: 37, HCDR2 shown in SEQ ID NO: 38 and HCDR3 shown in SEQ ID NO: 39,
(4) HCDR1 shown in SEQ ID NO: 40, HCDR2 shown in SEQ ID NO: 41 and HCDR3 shown in SEQ ID NO: 42, or variants of HCDR1 shown in SEQ ID NO: 40, HCDR2 shown in SEQ ID NO: 41 and HCDR3 shown in SEQ ID NO: 42, and a combination thereof, wherein the variants each have one or more (preferably one, two or three) conservative amino acid mutations (preferably a substitution, an insertion or a deletion) compared with HCDR1 shown in SEQ ID NO: 40, HCDR2 shown in SEQ ID NO: 41 and HCDR3 shown in SEQ ID NO: 42, respectively, and an amino acid sequence that has an affinity for binding to ANG2 is retained,
preferably, HCDR1 shown in SEQ ID NO: 40, HCDR2 shown in SEQ ID NO: 41 and HCDR3 shown in SEQ ID NO: 42, wherein amino acids at positions 5 to 8 and positions 10 and 11 in CDR1 shown in SEQ ID NO: 40, amino acids at positions 2 to 5 and position 7 in CDR2 shown in SEQ ID NO: 41 and amino acids at position 1 and positions 3 to 8 in CDR3 shown in SEQ ID NO: 42 are selected from an amino acid X, and the amino acid X is selected from a group consisting of Ala, Arg, Asn, Asp, Cys, Gln, Glu, Gly, His, Ile, Leu, Lys, Met, Phe, Pro, Ser, Thr, Trp, Tyr and Val,
preferably, a variant of HCDR1 shown in SEQ ID NO: 40, wherein glycine G at position 1 may be substituted by an amino acid selected from a group consisting of: Ala, Val, Leu and Ile; phenylalanine F at position 2 may be substituted by Tyr; proline P at position 3 may be substituted by an amino acid selected from a group consisting of: Trp and His; Ser at position 4 may be substituted by Thr; Ser at position 9 may be substituted by Thr; and Gln at position 12 may be substituted by Asn;
a variant of HCDR2 shown in SEQ ID NO: 41, wherein Ile at position 1 may be substituted by an amino acid selected from a group consisting of: Ala, Val, leu and Gly; Leu at position 6 may be substituted by Ile; and Lys at position 8 may be substituted by Arg;
a variant of HCDR3 shown in SEQ ID NO: 42, wherein Val at position 2 may be substituted by an amino acid selected from a group consisting of: Ala, Gly, Leu and Ile; and Asp at position 9 may be substituted by Glu,
(5) HCDR1 shown in SEQ ID NO: 43, HCDR2 shown in SEQ ID NO: 44 and HCDR3 shown in SEQ ID NO: 45,
(6) HCDR1 shown in SEQ ID NO: 46, HCDR2 shown in SEQ ID NO: 44 and HCDR3 shown in SEQ ID NO: 48,
(7) HCDR1 shown in SEQ ID NO: 49, HCDR2 shown in SEQ ID NO: 44 and HCDR3 shown in SEQ ID NO: 48,
(8) HCDR1 shown in SEQ ID NO: 52, HCDR2 shown in SEQ ID NO: 44 and HCDR3 shown in SEQ ID NO: 48, and
(9) HCDR1 shown in SEQ ID NO: 55, HCDR2 shown in SEQ ID NO: 56 and HCDR3 shown in SEQ ID NO: 57.

2. The anti-ANG2 heavy-chain single-domain antibody according to claim 1, wherein ANG2 is selected from human ANG2, rabbit ANG2 or monkey ANG2.

3. The anti-ANG2 heavy-chain single-domain antibody according to claim 1 or 2, comprising a heavy chain variable region, wherein the heavy chain variable region comprises or consists of a sequence shown in any one of SEQ ID NOs: 1 to 10 or a sequence having at least 80%, 85%, 86%, 87%, 88%, 89%, 90%, 91%, 92%, 93%, 94%, 95%, 96%, 97%, 98% or 99% homology with the sequence shown in any one of SEQ ID NOs: 1 to 10 and having ANG2 binding activity.

4. A recombinant protein, comprising the anti-ANG2 heavy-chain single-domain antibody according to any one of claims 1 to 3, preferably, the recombinant protein further comprises a bioactive substance, for example, a toxin having enzymatic activity or an active fragment (for example, abrin, ricin A, pseudomonas exotoxin or diphtheria toxin) of the toxin, a tumor necrosis factor or an interferon (for example, IFN-γ), a biological response modifier (for example, lymphokines IL-2, IL-2, IL2-6, IL-10 and GM-CSF) and an Fc fragment (for example, from an Fc region of IgG (for example, an IgG1, IgG2, IgG3 or IgG4 subtype), IgA1, IgA2, IgD, IgE or IgM, for example, from an Fc region of human, rabbit or monkey IgG, IgA1, IgD, IgE or IgM), preferably, the anti-ANG2 heavy-chain single-domain antibody and the Fc fragment are linked by a linker peptide, preferably, the linker peptide is (GGGGS)n, wherein n = 1, 2 or 3, preferably, the Fc fragment is a human IgG1 heavy chain constant region, more preferably an Fc fragment shown in GenBank No. AK303185.1, more preferably, the recombinant protein comprises or consists of sequences shown in SEQ ID NOs: 11 to 20.

5. A multi-specific antibody, preferably a bispecific antibody, comprising the anti-ANG2 heavy-chain single-domain antibody according to any one of claims 1 to 3, preferably, a structure of the bispecific antibody comprises a light chain and a heavy chain, wherein the light chain and the heavy chain are paired to form an interchain disulfide bond, and two heavy chains are paired to form an interchain disulfide bond, wherein the heavy chain is (VH)-(CH1)-(hinge region)-(Fc)-(linker peptide)-(VHH), and the light chain is (VL)-(light chain constant region).

6. The multi-specific antibody according to claim 5, wherein VHH is the anti-ANG2 single domain antibody according to any one of claims 1 to 3, VH and VL are heavy chain and light chain variable regions of a second antibody, preferably, an antigen targeted by the second antibody is selected from an immune cell surface antigen, a tumor antigen, a virus, a bacterium, an endotoxin, a cytokine or a combination thereof; more preferably, the antigen targeted by the second antibody is selected from PD-L1, PD-1, VEGFA, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, GD3, GM2, RANKL, CD3 and/or CD16a, preferably, the antigen targeted by the second antibody is VEGF, more preferably, the second antibody is selected from an anti-VEGF antibody, the anti-VEGF antibody comprises HCDR1, HCDR2 and HCDR3 contained in a heavy chain variable region shown in SEQ ID NO: 24 (preferably, according to an IMGT numbering system, the anti-VEGF antibody comprises HCDR1 shown in SEQ ID NO: 50, HCDR2 shown in SEQ ID NO: 51 and HCDR3 shown in SEQ ID NO: 53) and LCDR1, LCDR2 and LCDR3 contained in a light chain variable region shown in SEQ ID NO: 27 (preferably, according to the IMGT numbering system, the anti-VEGF antibody comprises LCDR1 shown in SEQ ID NO: 54, LCDR2 shown in SEQ ID NO: 58 and LCDR3 shown in SEQ ID NO: 59), more preferably, a heavy chain variable region of the anti-VEGF antibody comprises or consists of a sequence shown in SEQ ID NO: 24 or a sequence having at least 80%, 85%, 90%, 91%, 92%, 93%, 94%, 95%, 96%, 97%, 98% or 99% homology with the sequence shown in SEQ ID NO: 24, and a light chain variable region of the anti-VEGF antibody comprises or consists of a sequence shown in SEQ ID NO: 27 or a sequence having at least 80%, 85%, 90%, 91%, 92%, 93%, 94%, 95%, 96%, 97%, 98% or 99% homology with the sequence shown in SEQ ID NO: 27.

7. The multi-specific antibody according to claim 5 or 6, wherein a sequence of CH1 is shown in SEQ ID NO: 25, a sequence of the hinge region is shown in SEQ ID NO: 26, a sequence of the Fc constant region is shown in SEQ ID NO: 29, a sequence of the linker peptide is shown in SEQ ID NO: 30 or 47, and a sequence of the light chain constant region is shown in SEQ ID NO: 28.

8. A polynucleotide, encoding the anti-ANG2 heavy-chain single-domain antibody according to any one of claims 1 to 3, the recombinant protein according to claim 4 or the multi-specific antibody according to any one of claims 5 to 7.

9. A vector, comprising the polynucleotide according to claim 8.

10. A host cell, comprising the vector according to claim 9.

11. A conjugate, comprising the anti-ANG2 heavy-chain single-domain antibody according to any one of claims 1 to 3, the recombinant protein according to claim 4, or the multi-specific antibody according to any one of claims 5 to 7, and a conjugated moiety, wherein the conjugated moiety is a purification tag (for example, a His tag), a detectable label, a drug, a toxin, a cytokine, an enzyme or a combination thereof; preferably, the conjugated moiety is a radioisotope, a fluorescent substance, a chemiluminescent substance, a colored substance, a chemotherapeutic agent, a biological toxin, polyethylene glycol or an enzyme.

12. A kit, comprising the anti-ANG2 heavy-chain single-domain antibody according to any one of claims 1 to 3, the recombinant protein according to claim 4, the multi-specific antibody according to any one of claims 5 to 7 or the conjugate according to claim 11; preferably, the kit further comprises an anti-ANG2 heavy-chain single-domain antibody, a recombinant protein or a multi-specific antibody specifically recognizing the anti-ANG2 heavy-chain single-domain antibody according to any one of claims 1 to 3, the recombinant protein according to claim 4, the multi-specific antibody according to any one of claims 5 to 7 or the conjugate according to claim 11; optionally, the anti-ANG2 heavy-chain single-domain antibody, the recombinant protein or the multi-specific antibody further comprises a detectable label, for example, a radioisotope, a fluorescent substance, a chemiluminescent substance, a colored substance or an enzyme; preferably, the kit is configured to detect the presence or a level of ANG2 in a sample; or
the kit comprises: (1) the anti-ANG2 heavy-chain single-domain antibody according to any one of claims 1 to 3, the recombinant protein according to claim 4, the multi-specific antibody according to any one of claims 5 to 7 or the conjugate according to claim 11, and (2) an antibody or an antigen-binding fragment of the antibody that targets another antigen, and/or a cytotoxic agent, and/or a chemotherapeutic drug, and optionally, an instruction for use.

13. A pharmaceutical composition, comprising the anti-ANG2 heavy-chain single-domain antibody according to any one of claims 1 to 3, the recombinant protein according to claim 4, the multi-specific antibody according to any one of claims 5 to 7 or the conjugate according to claim 11, optionally, the pharmaceutical composition further comprises a pharmaceutically acceptable carrier and/or an excipient, preferably, the pharmaceutical composition is in a form suitable for administration through subcutaneous injection, intradermal injection, intravenous injection, intramuscular injection or intralesional injection.

14. Use of the anti-ANG2 heavy-chain single-domain antibody according to any one of claims 1 to 3, the recombinant protein according to claim 4, the multi-specific antibody according to any one of claims 5 to 7 or the conjugate according to claim 11 for inhibiting pro-angiogenesis activity of ANG2, preventing and/or treating diseases caused by or related to an angiogenic process such as fundus neovascular disease, rheumatoid arthritis and psoriasis, and/or a tumor, or use for preparing drugs for inhibiting the pro-angiogenesis activity of ANG2, preventing and/or treating the diseases caused by or related to the angiogenic process such as fundus neovascular disease, rheumatoid arthritis and psoriasis, and/or the tumor.
